# EUROPEAN PATENT APPLICATION

(11) **EP 3 633 028 A1**
(43) Date of publication of application: **08.04.2020**
(21) Application number: 18198244.8
(22) Date of filing: 02.10.2018
(51) Int. Cl.: C12N 5/0775, C12N 5/00

(54) **METHOD FOR PRODUCING A VIRUS-FREE PLATELET LYSATE MATERIAL**

(71) Applicant: PL BioScience GmbH, 52068 Aachen (DE)
(72) Inventor: HEMEDA, Hatim, 52511 Geilenkirchen (DE); WILKES, Christian, 48734 Reken (DE)
(74) Representative: Remus, Alvaro Johannes

(57) **Abstract**

The invention relates to a method for producing a material for preparing a substrate for cultivating living cells. The invention further relates to a platelet lysate material and its use. The solution of the problem underlying the invention is based on the combination of at least two different treatments with different virus inactivation procedures. Combining two or more procedures for inactivation of virus particles and/or virus components ensures that a wide range of viruses (e.g. enveloped and non-enveloped viruses) are inactivated so as to provide a safe product for clinical and pharmaceutical applications. Moreover, a platelet lysate material comprising blood platelet lysate is provided, wherein the material is a liquid or dry material that is essentially devoid of virus particles and/or virus components.

## Description

### Background of the invention

The invention basically relates to a method for producing a material for preparing a substrate for cultivating living cells. The invention further relates to a platelet lysate material and its use.

### Prior art

Cells are usually cultured in media that are supplemented with fetal calf serum (FCS) or other blood sera in order to provide the cells with essential growth factors. Although not any cell type actually needs serum for growth, most of the sensitive cell types, e.g., stem cells, are by now still dependent on serum-containing media. However, in light of therapeutic applications there is growing interest to avoid the use of FCS due to potential xenogeneic immune reactions (e.g. anti-FCS antibodies), bovine pathogens (viruses and prions) and a high lotto-lot variability that hampers reproducibility of results. Chemically defined synthetic culture media are now commercially available, but their precise composition is often undisclosed and they often include other xenogeneic components such as recombinant growth factors.

Due to the lack of any xenogeneic components, human platelet lysate (HPL) has been demonstrated to be the best suitable alternative for stem cell expansion, not only in basic research but also for clinical and therapeutic applications.

WO 2017/207688 A1 relates to a method for producing a material for preparing a three-dimensional, gel-like substrate, wherein a thrombocyte concentrate is provided and blood platelets are harvested from this thrombocyte concentrate. The blood platelets are then lysed so as to produce a platelet lysate, and said platelet lysate is subsequently lyophilized so as to provide a dry platelet lysate material.

The lyophilized material can be easily reconstituted by the addition of water at the place of use in order to provide a completely functional platelet lysate gel which can be used for cultivating living cells without any limitations. Accordingly, this method facilitates transportation of the gel material and ensures a higher shelf life of the platelet lysate gel without any impact on cell culture properties.

A method for preparing a lyophilized platelet lysate is known from US 2013/0195959 A1. This document discloses a method for preparing a composition suitable for therapeutic use or as a culture medium which comprises the steps of concentrating platelets from a platelet source to form a platelet rich portion of the platelet source, and lysing the platelets in the platelet rich portion to form a plurality of lysates. An additional step includes lyophilizing the lysates to form lyophilized platelet lysates in a composition with released concentrations of available growth factors, cytokines, and chemokines. These lyophilized platelet lysates can be applied in dry form or rehydrated prior to application, or as a gel.

Furthermore, WO 2013/076507 A2 discloses a pharmaceutical composition comprising a platelet lysate, and therapeutic uses thereof. The composition can be therapeutically applied to wounds. The platelet lysate is lyophilized to produce a stabilized, freeze-dried powder that contains growth factors and which can be used for therapeutic application in wound healing, but also other therapeutic applications in which there is an increased need for delivery of natural growth factors. The lyophilized platelet lysate can be resuspended in medium or saline, sterilized by filtration, and then used to produce a platelet-rich plasma gel.

However, although human platelet lysate is a commonly used supplement for culturing Mesenchymal Stem Cells (MSCs) that, in contrast to fetal calf serum, does not harbor the risk of xenogeneic immune reactions or transmission of bovine pathogens, viral contamination is still an issue in respect of clinical and pharmaceutical applications. Especially for any clinical applications based on human-derived ancillary materials, viral safety is one of the main aspects needed to be considered. When substrates for expanding stem cells or other therapeutically applicable cell types are prepared, it is therefore of paramount importance to reduce or completely eliminate any viral contamination risks.

### Summary of the invention

It is the object of the invention to provide a method for producing a material for preparing a substrate for cultivating living cells that completely eliminates or at least reduces any viral contamination risks.

The object is achieved by providing a method for producing a material for preparing a substrate for cultivating living cells, said method comprising:
- Providing a blood platelet lysate, and
- Exposing said platelet lysate to at least two different treatments, each treatment being capable of inactivating virus particles and/or virus components, so as to provide a virus-free or at least virus-reduced platelet lysate material.

The solution of the problem underlying the invention is based on the combination of at least two different treatments with different virus inactivation procedures. Combining two or more procedures for inactivation of virus particles and/or virus components ensures that a wide range of viruses (e.g. enveloped and non-enveloped viruses) are inactivated so as to provide a safe product for clinical and pharmaceutical applications. As each virus inactivating procedure typically inactivates only one or at most a few types of viruses, the combination of different inactivating procedures significantly increases the safety degree. Thus, by the method according to the invention, a safe platelet lysate material for expanding stem cells or other therapeutically applicable cell types without or at least significantly reduced viral contamination risk is provided.

In an advantageous embodiment of the invention the treatments comprise at least two different treatments selected from the group consisting of pasteurization, dry heat, vapour heat, irradiation with ultraviolet light, irradiation with gamma rays, irradiation with X-rays, electron-beam irradiation, nano-filtration, and solvent/detergent treatment. Basically, the choice of the treatment procedures depends on the virus type(s) to be eliminated (dsDNA, ssDNA, ssRNA etc.) and the composition of the material to be treated (high protein content, low protein content, protein-free etc.). However, in case of platelet lysate the treatments specified above turned out to be the most effective and mildest procedures for effective inactivation of virus particles and/or virus components.

Pasteurization: A process of treating a material with mild heat, i.e. less than 100°C, for example, heating biomolecules in solution, typically at 60 °C.

Dry heat: A process of heating a dry material, typically at 80 °C or higher, for example, following lyophilization.

Vapour heat: A process of heating a dry material and then reintroducing moisture preferably at 60 °C or at 80 °C, for example, following lyophilization.

UV irradiation: A process of applying ultraviolet light (UV) to a material, preferably at a wavelength of 100-280 nm (UVC) or 315-400 nm (UVA).

Gamma irradiation: A process of treating liquid, frozen or lyophilized material using irradiation with gamma rays for virus inactivation or bacterial sterilization. Gamma ray is a penetrating electromagnetic radiation arising from the radioactive decay of atomic nuclei and typically has wavelengths shorter than those of X-rays.

X-rays: Irradiation with X-rays (X rays, X-radiation, Röntgen radiation) is another form of electromagnetic radiation mostly at a wavelength ranging from 0.01 to 10 nanometers, corresponding to frequencies in the range 30 petahertz to 30 exahertz (3×10¹⁶ Hz to 3×10¹⁹ Hz) and energies in the range 100 eV to 100 keV.

Electron-beam irradiation: A process of treating a material with streams of electrons (cathode rays) emitted by a negative electrode (cathode) in a vacuum tube.

Nano-filtration: A membrane filtration-based process that uses nanometer sized through-pores that pass through a membrane which usually has effective pore sizes of 50 nm or less, preferably pore sizes from 1-10 nanometers, for separating solids from fluids (liquids or gases).

S/D treatment: Solvent/detergent (S/D) treatment refers to a process of treating a material in solution, usually with an organic solvent (e.g., tri(nbutyl) phosphate) and a detergent such as Tween 80 or Triton X-100.

The blood platelet lysate may be liquid, wherein the liquid platelet lysate is exposed to the treatments. Alternatively, the blood platelet lysate can be dried before the treatments, so as to provide a dry platelet lysate material, wherein the dry platelet lysate material is then exposed to the treatments.

In an advantageous embodiment of the invention the blood platelet lysate is dried by lyophilization. This advantageous step prior to the treatments includes lyophilizing the platelet lysate to form a dry, powder-like platelet lysate material. Surprisingly, the lyophilization step has no impact on the properties of the resulting substrate and provides a more stable supplement by increasing the shelf life for long time period under quality-controlled conditions. That is, the lyophilized platelet lysate material is stable and can be stored over a long period. Moreover, the lyophilized material can be easily transported so that the complete composition of the substrate can be delivered to the place of use. The user does not need to accomplish the whole process of producing a platelet lysate substrate at the place of use. Instead, the platelet lysate material is produced under standardized and controlled conditions in a production facility and the virus-free dry platelet lysate material is then delivered to the user as a kind of ready-to-use material which can be easily reconstituted by the user.

The advantage of this embodiment is based on the combination of a freeze-dried (by lyophilization) and virus-inactivated blood platelet lysate. The freeze-dried lysate can be stored at ambient temperature conditions instead of storage of the soluble form under frozen conditions (-20 degrees centigrade or below), wherein lyophilisation increases the stability of the material compared to the liquid state. It is an advantage of the method according to the invention that the freeze-dried human platelet lysate can be virus-inactivated in lower dose manner compared to the hydrated form. For example, according to the invention irradiation may be used for virus inactivation. The consequence of a lower irradiation dose is that less essential growth factors and other proteins of the human platelet lysate are damaged by irradiation. The performance of the human platelet lysate, e.g., in terms of cell growth is nonetheless affected, but it is significantly less affected compared to the higher dose irradiation needed to treat hydrated forms. Accordingly, a safer human platelet lysate product with higher performance compared to those which are conventionally virus-inactivated is provided. In general, lyophilization adds the benefit of preserving the growth factors, cytokines, chemokines, and other contents but also exhibits additional advantages in terms of storage, transport and logistics, while irradiation adds the advantage of effective inactivation of virus particles and/or virus components such as viral RNA and DNA with less damage of the platelet lysate's ingredients.

In total, a method for preparing a substrate for cultivating and expanding living cells suitable for therapeutic use and a virus-free or virus-reduced human Platelet Lysate (hPL) for clinical and therapeutic applications are provided. The method according to the invention provides a safe product for therapeutic applications without any impact on cell culture properties and facilitates storage and transport conditions with higher shelf life and stability without any impact on performance properties.

For the sake of convenience, the dry platelet lysate material can be ground, preferably prior to the treatments, so as to obtain a powder.

In another advantageous embodiment of the invention a liquid is added to the dry platelet lysate material, so as to obtain a reconstituted platelet lysate material. The virus-free dry platelet lysate material may be rehydrated by adding water, saline or liquid medium. For example, at least one medium component can be added to the rehydrated platelet lysate material so as to adapt the resulting substrate to the specific needs of the cells to be expanded. The medium component may comprise, for example, a standardized medium composition for cell culture, preferably Dulbecco's Modified Eagles medium (DMEM), in particular DMEM low glucose (DMEM-LG). Thus, a virus-free or at least virus-reduced substrate containing essential growth factors and all necessary nutrients for cell cultures without undesired serum components can be provided.

In a further advantageous embodiment of the invention the blood platelets are derived from thrombocyte concentrate and subsequently lysed by a freeze-thaw process. For example, the harvested platelet units may be frozen twice at -80°C and respectively re-thawed at 37°C.

The object is also achieved by providing a platelet lysate material comprising blood platelet lysate, said material being a liquid or dry material that is essentially devoid of virus particles and/or virus components. The virus-free or at least virus-reduced liquid material can be produced as a kind of ready-to-use material that can be directly used at the place of use The virus-free or at least virus-reduced dry material is stable and can be stored over a long period of time. Moreover, the dry material can be easily transported so that the complete substrate can be delivered to the place of use. The user does not need to accomplish the whole process of producing a platelet lysate substrate at the place of use. Instead, the virus-free or at least virus-reduced dry platelet lysate material is produced under standardized and controlled conditions in a production facility and then delivered to the user as a kind of ready-to-use material which can be easily reconstituted by the user.

For example, the platelet lysate material may comprise 1-50 % blood platelet lysate, preferably 1-40 % blood platelet lysate, more preferred 1-30 % blood platelet lysate, more preferred 1-20 % blood platelet lysate, more preferred 5-15 % blood platelet lysate, in particular approximately 10 % blood platelet lysate.

In an advantageous embodiment of the invention the blood platelets are derived from human blood. The use of human platelet lysate is especially beneficial for culturing human cells (e.g., human Mesenchymal Stem Cells) without the risk of xenogeneic immune reactions or transmission of (non-human) pathogens.

In a preferred embodiment of the invention the platelet lysate material is a powder.

Another aspect of the invention is the use of the platelet lysate material according to the invention, in particular the platelet lysate material produced in the method according to the invention, for preparing a virus-free or at least virus-reduced substrate for expanding living cells for clinical and/or therapeutic applications. In particular, the invention concerns the development of a safer type of (human) platelet lysate for therapeutic applications and a method for preparing a virus-free or at least virus-reduced substrate suitable for therapeutic use or as a cell culture medium for expanding living cells for clinical or therapeutic applications.

The produced virus-free or at least virus-reduced substrate can be a liquid substrate or a three-dimensional, gel-like substrate. The liquid or gel-like substrate according to the invention can advantageously be used as a pharmaceutical composition in cell therapy. Particularly, cells, preferably stem cells, in particular mesenchymal stromal cells (MSC), cultured in the liquid or embedded in the gel according to the invention may be transplanted into the body of a living animal or human for cell replacement, tissue repair, or the like. Alternatively, the substrate according to the invention may be transplanted and/or injected into an animal or human body in order to provide a suitable substrate for cells of the surrounding tissue or blood. The material according to the invention may be used for treating, e.g., arthrosis, necrosis, or other diseases correlated with damaged tissue.

The living cells to be cultivated or expanded may be, for example, mesenchymal stem cells. For example, the cells to be cultivated or expanded are stem or progenitor cells, particularly preferred mesenchymal stem cells, in particular mesenchymal stromal cells. In another exemplary embodiment of the invention, the cells are mesodermal cells, preferably fibroblasts or mesenchymal stromal cells. But, obviously, also other types of cells, for example, cells derived from ectodermal or endodermal lineages, or even primary cells, can be cultivated.

The term "lyophilization" as used herein refers to a "freeze-dry" process comprising the conversion of water from a frozen state to a gaseous state without going through a liquid state. The freeze-dry process removes moisture from a water-containing material while the material remains frozen. The basic process of lyophilization comprises the following steps: freezing, primary drying (sublimation), and secondary drying (desorption). At first, a dissolved and/or suspended substance is frozen at a low temperature (for example, -60 C°). Slow freezing produces larger crystals which allow the sublimating material to escape. Some products form a glassy material and annealing may be required during the freezing process. Annealing, first lowering the temperature then raising the temperature and then lowering it again, locks the constituents in place and then allows the crystals to grow. Freezing can range from 1 hour to 24 hours, depending on the application. In step 2 (primary drying) the water or diluent is then extracted via vacuum, resulting in a porous, dry "cake". Sublimation occurs under vacuum with the product temperature below its critical temperature. This is typically the longest process. At the end of the primary drying cycle, the product will have 3 to 5 % moisture content. There is a final drying step (secondary drying) to remove residual unfrozen water molecules. This is done by heating the product. Secondary drying can result in moisture levels of about 0.5 %.

The term "irradiation" as used herein refers to the treatment of material by applying any kind of radiation (rays) suitable for inactivation of virus particles or virus components to said material. The radiation may comprise, for example, gamma rays, heat rays, UV rays, X rays, and electron rays.

For example, the following viruses can be inactivated using the method according to the invention:
BEV (Bovine enterovirus): A non-enveloped, single-stranded RNA virus used as a model for hepatitis A virus.
BVDV (Bovine viral diarrhoea virus): An enveloped, single-stranded RNA virus used as a model for hepatitis C virus.
CMV (Cytomegalovirus): An enveloped, double-stranded DNA virus, typically cell-associated.
Coxsackie virus: A non-enveloped, single-stranded RNA virus.
CPV (Canine parvovirus): A non-enveloped, single-stranded DNA virus.
EBV (Epstein-Barr virus): An enveloped, double-stranded DNA virus, typically cell-associated.
EMCV (Encephalomyocarditis virus): A non-enveloped, single-stranded, RNA virus.
HAV (Hepatitis A virus): A non-enveloped, single-stranded RNA virus.
HBV (Hepatitis B virus): An enveloped, double-stranded DNA virus.
HCV (Hepatitis C virus): An enveloped, single-stranded, RNA virus.
HDV (Hepatitis delta virus): A defective virus which requires co-infection by hepatitis B virus.
HIV (Human immunodeficiency virus): An enveloped, single-stranded RNA virus.
HSV (Herpes simplex virus): An enveloped, double-stranded DNA virus, typically cell-associated.
HTLV 1 and 2 (Human T-cell lymphotropic virus, types 1 and 2): Enveloped, single-stranded RNA viruses, typically cell-associated.
Polio virus: A non-enveloped, single-stranded, RNA virus.
PPRV (Porcine pseudorabies virus): An enveloped, double-stranded DNA virus.
PPV (Porcine parvovirus): A non-enveloped, single-stranded DNA virus.
PRV (Pseudorabies virus): An enveloped, double-stranded DNA virus.
Sindbis virus: An enveloped, single-stranded RNA virus.
SLFV (Semliki forest virus): An enveloped, single-stranded, RNA virus.
Vaccinia virus: An enveloped, double-stranded DNA virus.
Vesicular stomatitis virus: An enveloped, single-stranded RNA virus.
West Nile virus: An enveloped, single-stranded RNA virus.

However, any other virus may also be inactivated by the method according to the invention.

The invention is further described in detail with reference to the examples.

### Description of exemplary and preferred embodiments of the invention

Mesenchymal stromal cells (MSCs) are a biologically important cell population that is able to support hematopoiesis, can differentiate along mesenchymal and nonmesenchymal lineages *in vitro,* is capable of suppressing alloresponses, and appears to be nonimmunogenic. These properties suggest emerging roles for mesenchymal stromal cells in cell therapy.

MSC were isolated from the caput femoris upon hip fracture after written consent using guidelines approved by the Ethic Committee of the University of Aachen. Bone fragments were flushed with PBS and mononuclear cells (MNC) subsequently isolated by a density gradient on Biocoll (Biochrom AG, Berlin, Germany). Cells were cultured in fibronectin coated cell culture dishes (Greiner, Kremsmünster, Austria) in Dulbecco's Modified Eagles Medium-Low Glucose (DMEM-LG; PAA, Pasching, Austria) with 2 mM L-glutamine (Sigma Aldrich, St. Louis, MO, USA) and 100 U/mL penicilline/streptomycine (pen/strep; Lonza, Basel, Switzerland). Culture medium was supplemented with FCS (HyClone, Bonn, Germany) or HPL as indicated. Culture medium was always changed twice per week. When reaching 80% confluence cells were trypsinized, counted by in a Neubauer counting chamber (Brand, Wertheim, Germany) and replated at 10⁴ cells/cm². Cumulative population doublings were calculated from the first passage onward.

Human platelet lysate (HPL) was generated from platelet units of healthy donors as described by Horn et al. (2010). In particular, HPL was generated by apheresis using the Trima Accel collection system (CaridianBCT, Garching, Germany). Such thrombocyte concentrates had a platelet content of 2.0 to 4.2×10¹¹ platelets in 200 mL plasma supplemented with Acid-Citrate-Dextrose (ACD) (1:11 v/v). Platelet units were aliquoted, twice frozen at -80°C, re-thawed at 37°C and centrifuged at 2600 g for 30 min at 4°C to remove cell fragments and pooled in equal amounts. The supernatant was filtered through 0.22 µm GD/X PVDF filter device (Whatman, Dassel, Germany), optionally supplemented with 2 U/mL heparin to avoid gelatinization, and stored at -80°C.

Cells were cultured in cell culture dishes (Greiner, Kremsmünster, Austria) in Dulbecco's Modified Eagles Medium-Low Glucose (DMEM-LG; PAA) with 2 mM L-glutamine (Sigma Aldrich, St. Louis, MO, USA) and 100 U/mL penicillin/streptomycin (pen/strep; Lonza, Basel, Switzerland). Culture medium/substrate was supplemented with HPL as indicated in the specification. Cells were cultured at 37°C in a humidified atmosphere containing 5% carbon dioxide with medium changes twice per week.

Cell proliferation was evaluated via the Thiazolyl Blue Tetrazolium Bromide (MTT) assay. MSC were seeded on a 96-well plate (3,000 cells/well) in culture medium/substrate supplemented with 0, 1, 5, 10, or 20% of HPL as indicated in the specification. After 7 days of culture, cells were washed with PBS (PAA) and incubated with 1 mM MTT (Sigma-Aldrich) in PBS for 3.5 h. The excess solution was discarded and crystals were resolved in 4 mM HCI in isopropanol (both from Roth, Karlsruhe, Germany). Optical density (OD) was measured at wave length 590 nm with a Tecan Infinite 2000 plate reader (Tecan Trading, Männedorf, Switzerland).

### Example

### Generation of human platelet lysate:

Platelet units were kindly provided by the Institute for Transfusion Medicine, RWTH Aachen University Medical School after their expiry date (5 days after harvesting). Platelet units were generated by apheresis using the Trima Accel Collection System (CaridianBCT, Garching, Germany) and comprise 2.0-4.2 × 10¹¹ platelets in 200 mL plasma supplemented with Acid-Citrate-Dextrose (ACD) (1:11 v/v). Aliquots of 45 mL were twice frozen at -80°C and re-thawed at 37°C and then centrifuged at 2,600 × g for 30 minutes. The supernatant was filtered through 0.2 µm GD/X PVDF filters (Whatman, Dassel, Germany) and stored at -80°C until use. Experiments of this study were performed with three HPL-pools each consisting of lysates of four donors but similar results were also observed using individual donor derived HPLs. HPLs consisted predominately of blood type A but a systematic analysis did not reveal any impact of the blood group.

### Culture medium and HPL-gel:

Culture medium consisted of Dulbecco's Modified Eagles Medium-Low Glucose (DMEM-LG; PAA, Pasching, Austria) with 2 mM L-glutamine (Sigma Aldrich, St. Louis, MO, USA) and 100 U/mL penicillin/streptomycin (pen/strep; Lonza, Basel, Switzerland) and HPL (10% if not indicated otherwise). Cells were cultured on tissue culture plastic (TCP; Greiner, Kremsmünster, Austria). For gel preparation, HPL containing cell culture medium without heparin was filled in cell culture plates. Due to the calcium comprised in the medium the coagulation cascade was initiated and the HPL-medium gelatinized within one hour at 37°C. That is, the blood platelet lysate, either pure or diluted, spontaneously gelatinizes at approximately 37 °C without the necessity to add any coagulation-inducing agent such as, for example, thrombin and/or calcium gluconate. The resulting HPL-gel was used as cell culture substrate which was seeded with the same amount of MNC. For comparison, gels consisting of 80% collagen G (3 mg/mL collagen type I/III in 12 mM HCI; Biochrome, Berlin, Germany) were used as described before. Cells were cultured at 37°C in 6 well plates or T75 tissue culture flasks (Nunc Thermo Fisher Scientific, Langenselbold, Germany).

### Isolation of human mesenchymal stromal cells from bone marrow:

MSC were isolated from mononuclear cells (MNC) by plastic adherence. In brief, bone fragments from the *caput femoris* of patients undergoing femoral head prosthesis were flushed with phosphate-buffered saline (PBS). Subsequently, MNC were isolated by Ficoll density gradient centrifugation (Biocoll Separating Solution, density 1.077g/l, Biochrom AG, Berlin, Germany) and washed twice with PBS. At least 5 × 10⁵ MNC were then resuspended in culture medium either on TCP, HPL-gel or collagen-gels in 6-well plates. Cells were cultured at 37°C in a humidified atmosphere with 5% CO₂. The first medium exchange was performed after 48 hours to remove non-adherent cells. Thereafter, half-medium changes were performed twice per week and MSC were further passaged as described below.

### Lyophilization:

Freezing is done by placing the gel in a freeze-drying flask and rotating the flask in a shell freezer which is cooled by mechanical refrigeration, dry ice and methanol, or liquid nitrogen. On a larger scale, freezing can be accomplished using a freeze-drying machine. In this step, it is important to cool the material below its triple point, the lowest temperature at which the solid and liquid phases of the material can coexist. This ensures that sublimation rather than melting will occur in the following steps. Larger crystals are easier to freeze-dry. To produce larger crystals, the product should be frozen slowly or can be cycled up and down in temperature (annealing). The freezing temperatures are between -50 C and -80 C.

During the primary drying phase, the pressure is lowered (to the range of a few millibars), and enough heat is supplied to the frozen gel for the ice to sublime. In this initial drying phase, about 95% of the water in the material is sublimated. This phase may be slow because, if too much heat is added, the material's structure could be altered. In this phase, pressure is controlled through the application of partial vacuum. The vacuum speeds up the sublimation, making it useful as a deliberate drying process. Furthermore, a cold condenser chamber and/or condenser plates provide a surface for the water vapour to re-solidify on. Condenser temperatures are typically below -50 C (-60 F).

The secondary drying phase aims to remove unfrozen water molecules, since the ice was already removed in the primary drying phase. In this phase, the temperature is raised higher than in the primary drying phase, and can even be above 0 °C, to break any physico-chemical interactions that have formed between the water molecules and the frozen material. The pressure can also be lowered in this stage to encourage desorption (for example, in the range of microbars, or fractions of a pascal).

After the freeze-drying process is complete, the vacuum is broken with an inert gas, such as nitrogen, before the material is sealed. At the end of the operation, the final residual water content in the product is extremely low, around 1% to 4%.

### Non-patent literature

Horn, P.; Bokermann, G.; Cholewa, D.; Bork, S.; Walenda, T.; Koch, C.; Drescher, W.; Hutschenreuther, G.; Zenke, M.; Ho, A.; Wagner, W.: Comparison of Individual Platelet Lysates for Isolation of Human Mesenchymal Stromal Cells. Cytotherapy 12: 888-898; 2010.

## Claims

1. A method for producing a material for preparing a substrate for cultivating living cells, said method comprising:
- Providing a blood platelet lysate, and
- Exposing said platelet lysate to at least two different treatments, each treatment being capable of inactivating virus particles and/or virus components, so as to provide a virus-free or at least virus-reduced platelet lysate material.

2. Method according to claim 1, wherein the treatments comprise at least two different treatments selected from the group consisting of pasteurization, dry heat, vapour heat, irradiation with ultraviolet light, irradiation with gamma rays, irradiation with X-rays, electron-beam irradiation, nano-filtration, and solvent/detergent treatment.

3. Method according to claim 1 or 2, wherein said blood platelet lysate is liquid, said liquid platelet lysate being exposed to the treatments.

4. Method according to claim 1, 2 or 3, wherein said blood platelet lysate is dried before the treatments, so as to provide a dry platelet lysate material, said dry platelet lysate material being exposed to the treatments.

5. Method according to claim 4, wherein the blood platelet lysate is dried by lyophilization.

6. Method according to claim 4 or 5, wherein the dry platelet lysate material is ground, so as to obtain a powder.

7. Method according to claim 4, 5 or 6, wherein a liquid is added to the dry platelet lysate material, so as to obtain a reconstituted platelet lysate material.

8. Method according to any one of claims 1 to 7, wherein the blood platelets are derived from thrombocyte concentrate and subsequently lysed by a freeze-thaw process.

9. A platelet lysate material comprising blood platelet lysate, said material being a liquid or dry material that is essentially devoid of virus particles and/or virus components.

10. The platelet lysate material according to claim 9 comprising 1-50 % blood platelet lysate, preferably 1-40 % blood platelet lysate, more preferred 1-30 % blood platelet lysate, more preferred 1-20 % blood platelet lysate, more preferred 5-15 % blood platelet lysate, in particular approximately 10 % blood platelet lysate.

11. The platelet lysate material according to claim 9 or 10, wherein the blood platelets are derived from human blood.

12. The platelet lysate material according to claim 9, 10 or 11, wherein said material is a powder.

13. Use of the platelet lysate material according to any one of claims 9 to 12, in particular the platelet lysate material produced in the method according to any one of claims 1 to 8, for preparing a virus-free or at least virus-reduced substrate for expanding living cells for clinical and/or therapeutic applications.

14. Use according to claim 13, wherein said substrate is a liquid substrate or a three-dimensional, gel-like substrate.

15. Use according to claim 13 or 14, wherein the cells are mesenchymal stem cells.
